# EUROPEAN PATENT SPECIFICATION

(11) **EP 4 349 286 B1**
(45) Date of publication and mention of the grant of the patent: **22.07.2026**
(21) Application number: 22815720.2
(22) Date of filing: 05.04.2022
(51) Int. Cl.: A61B 17/34

(54) **OBTURATOR AND TROCAR**
OBTURATOR UND TROKAR
OBTURATEUR ET TROCART

(30) Priority: 31.05.2021 JP 2021091900
(43) Date of publication of application: 10.04.2024
(73) Proprietor: FUJIFILM Corporation, Tokyo 106-8620 (JP)
(72) Inventor: TANAKA, Kunihiko, Ashigarakami-gun, Kanagawa 258-8538 (JP)
(74) Representative: Dehns Germany Partnerschaft mbB
(86) International application number: PCT/JP2022/017108
(87) International publication number: WO 2022/254964

(56) References cited:
- WO-A1-2021/015398
- JP-A- 2012 505 027
- JP-A- H05 245 154
- JP-A- H06 142 111
- JP-A- H09 253 087
- US-A1- 2003 191 414
- US-A1- 2005 077 689
- US-A1- 2010 274 096
- US-A1- 2019 216 497
- US-A1- 2021 128 133

## Description

### BACKGROUND OF THE INVENTION

### 1. Field of the Invention

The present disclosed technology relates to an obturator and a trocar.

### 2. Description of the Related Art

In surgery, a trocar is used. The trocar is composed of a cannula and an obturator. The cannula has a tubular shape with an insertion hole through which an endoscope and/or a treatment tool is guided into a body cavity. The obturator includes a shaft that is inserted into the insertion hole of the cannula and of which a distal end is sharp-pointed in a needle-like shape to be stuck into a body wall of a patient and a grip to which a proximal end of the shaft is attached and that is gripped by an operator. The operator separates the obturator from the cannula after sticking the shaft of the obturator into the body wall together with the cannula and then leaves the cannula alone in the body wall.

Described in JP2003-047618A is an obturator in which one recessed portion is formed at one surface of a quadrangular frustum-shaped grip. WO 2021/015398, over which the independent claim is characterised, discloses a disposable endoscopic cannula. US 2019/0216497 discloses a medical instrumenting a canula in communication with a trocar. US 2021/128133 discloses an organ retractor including a plurality of retractor jaws.

### SUMMARY OF THE INVENTION

In the case of the obturator disclosed in JP2003-047618A, an operator can operate the obturator with a thumb hooked on the recessed portion of the grip. However, since only one recessed portion is formed at the one surface of the grip, there is room for improvement in terms of operation stability.

An embodiment according to the present disclosed technology provides an obturator and a trocar improved in operation stability.

According to an aspect of the present invention, there is provided an obturator as claimed in claim 1.

The recessed portion includes at least a first surface that is formed along an axial direction of the shaft and a second surface that is connected to the first surface on a connection end side and that intersects the first surface, the connection end being one end of the grip to which the proximal end of the shaft is attached.

The first surface and the second surface are connected to each other by a curved surface.

It is preferable that at least one of the first surface or the second surface includes a curved surface.

It is preferable that the first surface extends from a position near the connection end to an open end, which is the other end of the grip and is on a side opposite to the connection end.

It is preferable that the grip has a columnar shape.

It is preferable that a length of the grip in an axial direction of the shaft is 1.2 times or more and less than 1.5 times a length of the grip in a radial direction of the shaft.

It is preferable that a length of the grip in an axial direction of the shaft is 40 mm or greater and smaller than 70 mm.

It is preferable that the grip includes a notched portion in which at least a portion of a button for separation of the cannula is accommodated and that is disposed at a position different from a position of the recessed portion in a circumferential direction of the shaft.

It is preferable that the grip includes a protrusion that is connected to the cannula and that is provided at a position corresponding to the recessed portion in a circumferential direction of the shaft.

A trocar according to an aspect of the present invention includes any obturator described above and a cannula into which the obturator is attachably and detachably inserted.

It is preferable that the cannula includes a cannula body that has a tubular shape with an insertion hole into which the shaft is inserted and a connecting portion to which a proximal end of the cannula body is attached, to which the grip is attachably and detachably connected, and of which a length in an axial direction of the shaft is shorter than a length of the grip in the axial direction.

According to the present disclosed technology, it is possible to provide an obturator and a trocar improved in operation stability.

### BRIEF DESCRIPTION OF THE DRAWINGS

Fig. 1 is a perspective view of a trocar.
Fig. 2 is an exploded perspective view of the trocar.
Fig. 3 is a plan view of the trocar.
Fig. 4 shows a side view and a front view of an obturator.
Fig. 5 is a view showing a state immediately before a shaft of the obturator is stuck into a body wall together with a cannula.
Fig. 6 is a view showing a state where the obturator is separated from a cannula and the cannula is left alone at a body wall.
Fig. 7 is a flowchart showing the procedure for operating the trocar.
Fig. 8 is a view showing a grip including a first surface composed of a curved surface protruding inward.
Fig. 9 is a view showing a grip including a first surface composed of a curved surface protruding outward.
Fig. 10 is a view showing a grip including a second surface composed of a curved surface protruding toward a connection end.
Fig. 11 is a view showing a grip including a first surface that is inclined to become closer to an inner side toward the connection end.
Fig. 12 is a view showing a grip including a deep cut surface obtained by deeply cutting a connection portion between a first surface and a second surface inwardly.
Fig. 13 is a view showing a grip having a truncated conical shape.
Fig. 14 is a view showing a truncated conical shape grip that includes a first surface composed of a curved surface protruding inward.
Fig. 15 is a view showing a truncated conical grip that includes a first surface extending from a position near the connection end to an open end.
Fig. 16 is a view showing a columnar grip.
Fig. 17 is a view showing a columnar grip including a first surface composed of a curved surface protruding inward.
Fig. 18 is a view showing another example of a grip.

### DESCRIPTION OF THE PREFERRED EMBODIMENTS

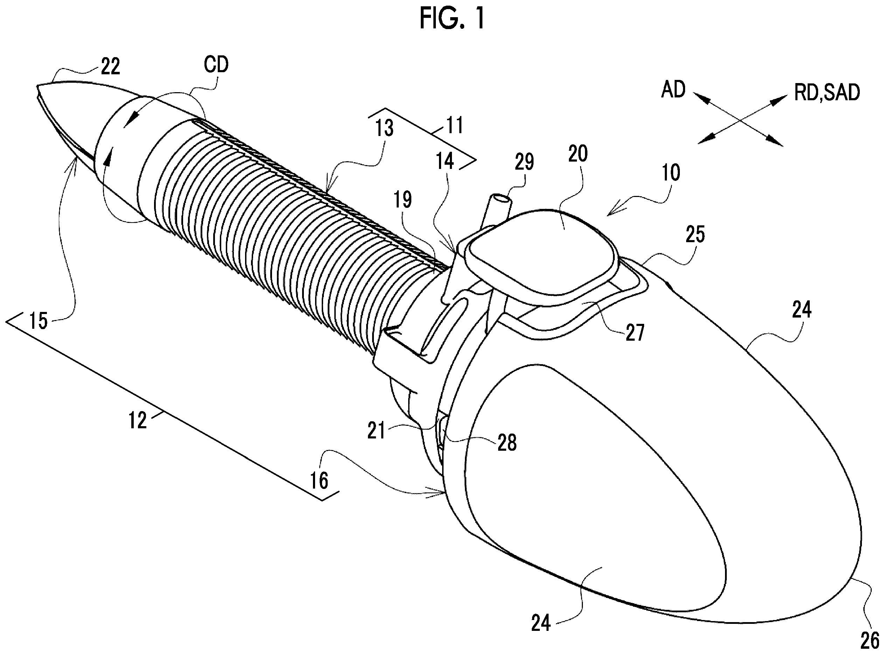

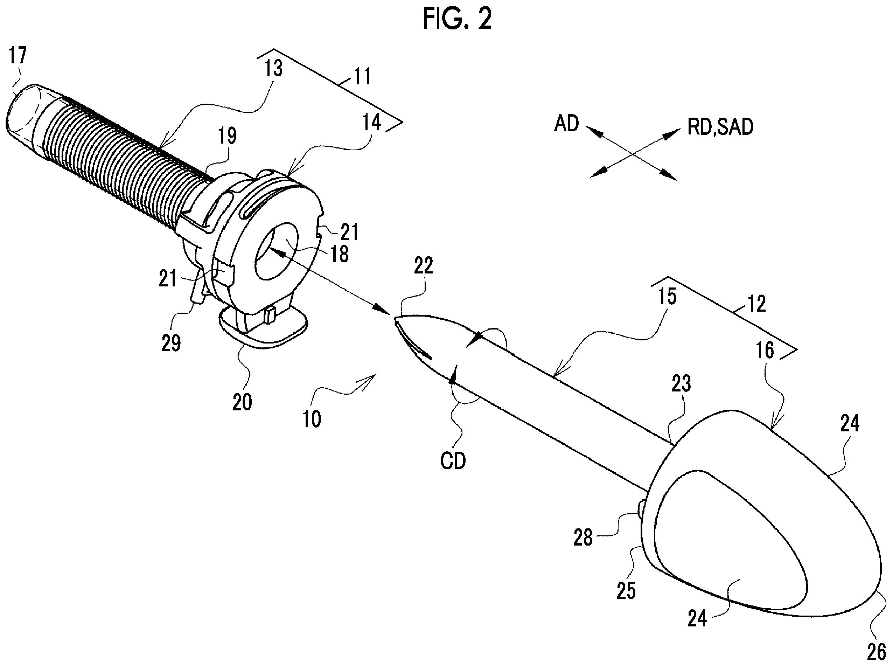

For example, as shown in Figs. 1 and 2, a trocar 10 is composed of a cannula 11 and an obturator 12. The cannula 11 includes a cannula body 13 and a connecting portion 14. The obturator 12 includes a shaft 15 and a grip 16. Note that Fig. 1 shows a state where the cannula 11 is mounted on the obturator 12 and Fig. 2 shows a state where the cannula 11 is separated from the obturator 12.

The cannula body 13 has a tubular shape with an insertion hole 17 into which the shaft 15 is inserted. The connecting portion 14 also has a tubular shape with an insertion hole 18 that leads to the insertion hole 17. The insertion holes 17 and 18 have substantially the same diameter as each other. The connecting portion 14 has a diameter slightly larger than the diameter of the cannula body 13 and is attached to a proximal end 19 of the cannula body 13. The grip 16 is attachably and detachably connected to the connecting portion 14.

The connecting portion 14 is provided with a button 20. The button 20 is operated by an operator in a case where the cannula 11 is to be separated from the obturator 12. In addition, at the connecting portion 14, two grooves 21 are formed along an axial direction AD of the shaft 15. The two grooves 21 are disposed at positions 180° separated from each other, that is, at positions facing each other in a plan view of the connecting portion 14 as seen in the axial direction AD of the shaft 15. Furthermore, the connecting portion 14 is provided with a port 29 through which a gas such as carbon dioxide is introduced into a body cavity. Although illustration and detailed description are omitted, the connecting portion 14 includes a latching mechanism for attachably and detachably holding the grip 16, a seal for preventing a gas and liquid in a body cavity from leaking to the outside, and the like. Note that a body cavity is, for example, an abdominal cavity.

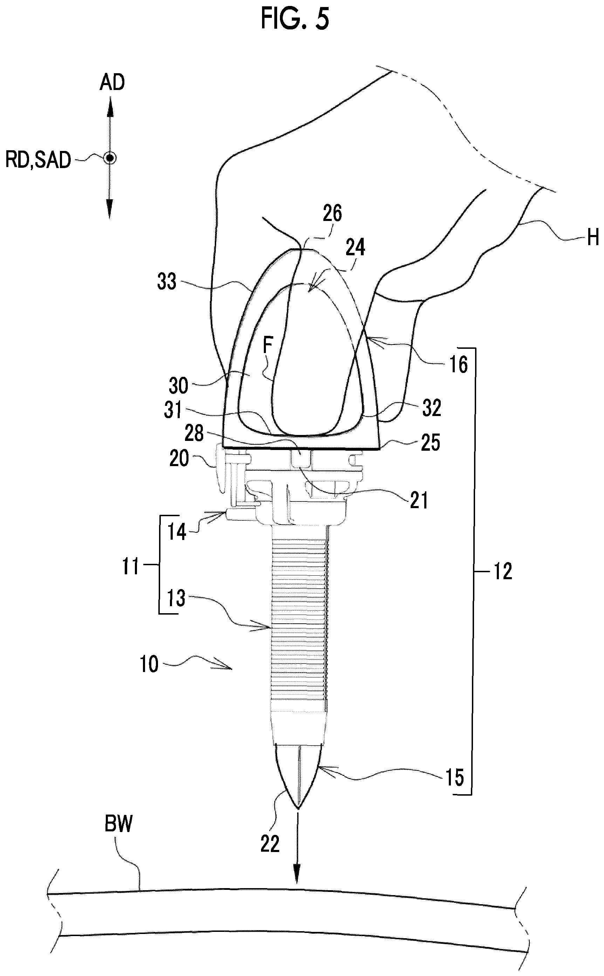

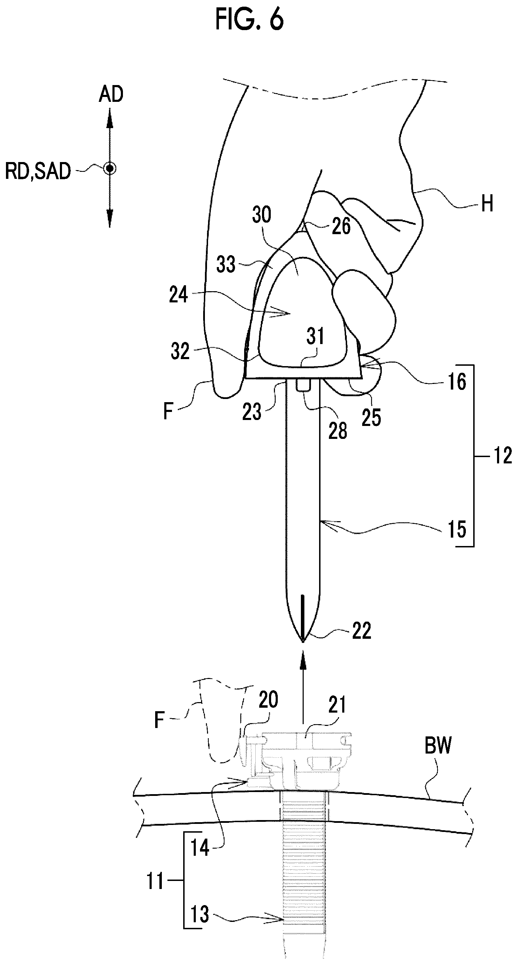

The shaft 15 has an elongated rod-like shape with a distal end 22 and a proximal end 23 and is made of, for example, metal, such as a titanium alloy. The distal end 22 is sharp-pointed in a needle-like shape to be stuck into a body wall BW (refer to Figs. 5 and 6) of a patient. The length of the shaft 15 in the axial direction AD is larger than the length of the cannula body 13 in the axial direction AD. Therefore, in a state where the cannula 11 is mounted on the obturator 12, the distal end 22 of the shaft 15 protrudes from the cannula body 13 as shown in Fig. 1. Note that the shaft 15 may be made of a resin, such as an acrylonitrile-butadienestyrene (ABS) resin. In addition, regarding the shaft 15, the distal end 22 may be made of an ABS resin and a portion other than the distal end 22 may be made of a different material, such as stainless steel, for example.

The grip 16 is attached to the proximal end 23 of the shaft 15. A corner of the grip 16 is rounded through R chamfering. The grip 16 is to be gripped by the operator (refer to Figs. 5 and 6). Two recessed portions 24 are formed at the grip 16. Each of the two recessed portions 24 is formed over an area from a position near a connection end 25, which is one end of the grip 16 and to which the proximal end 23 of the shaft 15 is attached, to a position near an open end 26, which is the other end of the grip 16 and is on a side opposite to the connection end 25. The two recessed portions 24 are disposed at positions 180° separated from each other, that is, at positions facing each other in a plan view of the grip 16 as seen in the axial direction AD of the shaft 15. Fingers of the operator are to be hooked on the recessed portions 24 (refer to Figs. 5 and 6). Therefore, the recessed portions 24 can also be referred to as finger hooking portions. Note that the operator refers to all persons who operate the trocar 10, such as a doctor who performs surgery using the trocar 10.

The connection end 25 is provided with a notched portion 27. Approximately half of the button 20 is accommodated in the notched portion 27. The notched portion 27 is disposed at a position different from the positions of the recessed portions 24 in a circumferential direction CD of the shaft 15. Specifically, the notched portion 27 is disposed at an intermediate position between the two recessed portions 24. In other words, in a plan view of the grip 16 as seen in the axial direction AD of the shaft 15, the notched portion 27 is disposed at a position 90° separated from each of the two recessed portions 24 which are disposed at the positions 180° separated from each other.

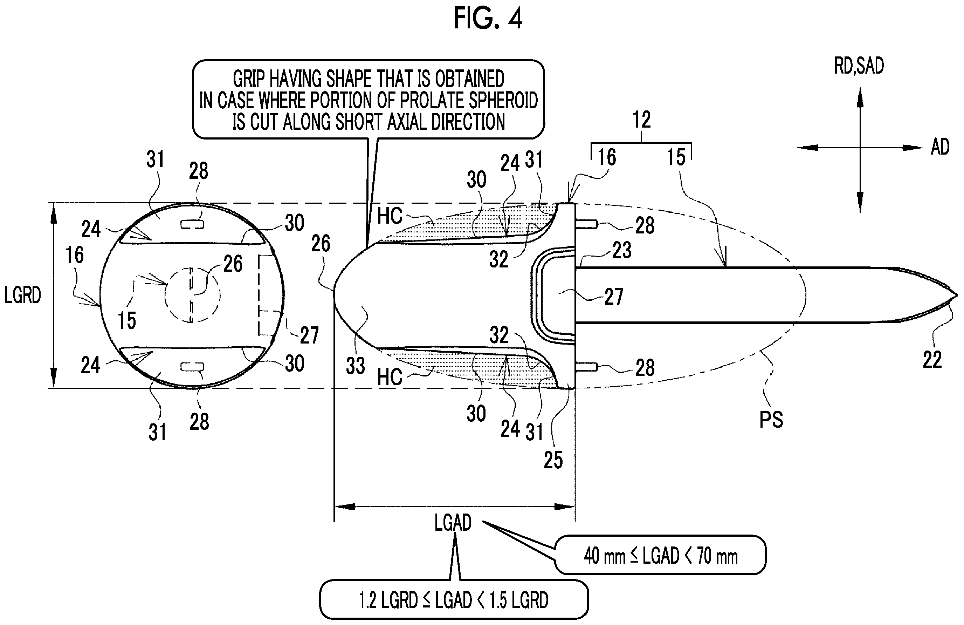

In addition, the connection end 25 is provided with two protrusions 28 (refer to Fig. 4 as well). As with the recessed portions 24, the two protrusions 28 are disposed at positions 180° separated from each other, that is, at positions facing each other in a plan view of the grip 16 as seen in the axial direction AD of the shaft 15. That is, the two protrusions 28 are provided at positions corresponding to the recessed portions 24 in the circumferential direction CD of the shaft 15. In a case where the cannula 11 is to be mounted on the obturator 12, the positions of the cannula 11 and the obturator 12 in the circumferential direction CD are adjusted such that the protrusions 28 are fitted into the grooves 21. That is, the grooves 21 and the protrusions 28 have a role of positioning the cannula 11 in the circumferential direction CD while the cannula 11 is being mounted on the obturator 12.

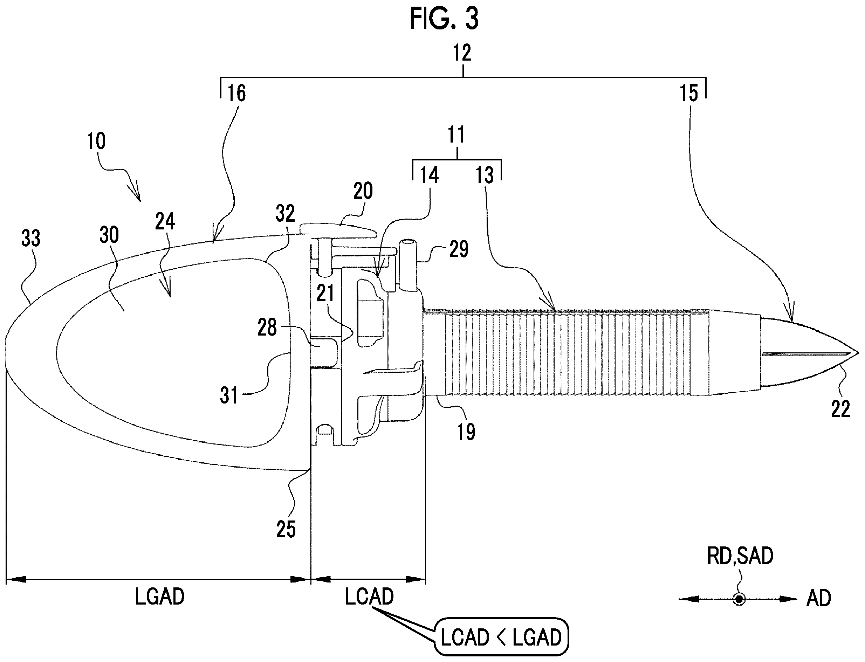

For example, as shown in Figs. 3 and 4, each of the recessed portions 24 includes a first surface 30 and a second surface 31. The first surface 30 is formed along the axial direction AD. Specifically, the first surface 30 is a surface approximately parallel to the axial direction AD. In contrast, the second surface 31 is formed along a radial direction RD of the shaft 15. Specifically, the second surface 31 is a surface approximately parallel to the radial direction RD. That is, the first surface 30 and the second surface 31 intersect each other. The second surface 31 is connected to the first surface 30 via a connection surface 32 on the connection end 25 side. The connection surface 32 is a curved surface that smoothly connects the first surface 30 and the second surface 31 to each other, and is an example of a "curved surface" according to the present disclosed technology. Although there are a plurality of radial directions RD, here, the radial direction RD refers to a direction orthogonal to the first surface 30.

The grip 16 has a tapered shape of which the thickness decreases toward the open end 26 from the connection end 25. More specifically, as shown in Fig. 4, the grip 16 has a shape that is obtained in a case where a portion (here, approximately half) of a prolate spheroid PS virtually represented by a two-dot chain line is cut along a short axial direction SAD which is the same direction as the radial direction RD. In the following description, a portion of the grip 16 that is on the open end 26 side, that is approximately half of the grip 16, and that includes a tapered curved surface will be referred to as a tapered portion 33. Note that the prolate spheroid PS is a three-dimensional shape obtained in a case where an ellipse is rotated with the major axis as the rotation axis and a rugby ball is a representative example thereof.

As shown in hatched areas HC in Fig. 4, each of the recessed portions 24 is formed by cutting or scraping off a portion of the grip 16 having a shape that is obtained in a case where approximately half of the prolate spheroid PS is cut along the short axial direction SAD. Therefore, the recessed portions 24 can also be referred to as cutting portions or scraped portions. In addition, the recessed portions 24 are level differences formed by cutting or scraping off a portion of the grip 16. Therefore, the recessed portions 24 can also be referred to as level difference portions.

As shown in Fig. 3, a length LCAD of the connecting portion 14 in the axial direction AD is shorter than a length LGAD of the grip 16 in the axial direction AD (LCAD < LGAD). In addition, as shown in Fig. 4, the length LGAD of the grip 16 in the axial direction AD is 1.2 times or more and less than 1.5 times a length LGRD of the grip 16 in the radial direction RD (1.2 LGRD ≤ LGAD < 1.5 LGRD). Furthermore, the length LGAD of the grip 16 in the axial direction AD is 40 mm or greater and smaller than 70 mm (40 mm ≤ LGAD < 70 mm). For example, the length LGAD of the grip 16 in the axial direction AD is 57 mm, and the length LGRD of the grip 16 in the radial direction RD is 42 mm. In this case, since LGAD ≈ 1.36 LGRD, a condition that 1.2 LGRD ≤ LGAD < 1.5 LGRD is satisfied. In addition, the above-described condition that 40 mm ≤ LGAD < 70 mm is also satisfied.

For example, as shown in Fig. 5, in a case where the trocar 10 is to be operated, the operator hooks fingers F of a hand H on the recessed portions 24 of the grip 16. Fig. 5 shows a case where a thumb is hooked on one recessed portion 24 and a finger other than the thumb is hooked on the other recessed portion 24. More specifically, the tip of the thumb is brought into contact with the second surface 31 of the one recessed portion 24 and the pad of the thumb is brought into contact with the first surface 30 and the connection surface 32 of the one recessed portion 24. In addition, the tip of at least one of fingers other than the thumb (for example, a middle finger) is brought into contact with the second surface 31 of the other recessed portion 24 and the pad of the finger other than the thumb is brought into contact with the first surface 30 and the connection surface 32 of the other recessed portion 24.

Fig. 5 shows a state immediately before the shaft 15 is stuck into the body wall BW together with the cannula 11. The operator causes the trocar 10 to access the body wall BW by bringing the trocar 10 down in an approximately perpendicular direction and, in some cases, the operator sticks the shaft 15 into the body wall BW up to a position where the connecting portion 14 reaches a surface of the body wall BW while rotating the trocar 10 in the circumferential direction CD. A portion of the body wall BW into which the shaft 15 is stuck may be incised in advance with a scalpel. Note that the body wall BW is, for example, an abdominal wall.

Fig. 6 shows a state where the cannula 11 is left alone at the body wall BW after the shaft 15 is stuck into the body wall BW up to a position where the connecting portion 14 reaches the surface of the body wall BW and then the obturator 12 is separated from the cannula 11. In this case, the operator switches from a gripping method as shown in Fig. 5 to a gripping method of wrapping the grip 16 (particularly, the tapered portion 33) with a palm of a hand as shown in the drawing. More specifically, the operator puts a thumb at a position between the two recessed portions 24 at which the button 20 is disposed, wraps an index finger, a middle finger, and a ring finger around the other recessed portion 24 side, and folds the index finger, the middle finger, and the ring finger. Furthermore, a little finger is folded along with the ring finger. The operator operates the button 20 with the tip of the thumb as represented by a broken line to disconnect the connecting portion 14 and the grip 16 from each other, that is, to disconnect the cannula 11 and the obturator 12 from each other. Then, the operator separates the obturator 12 from the cannula 11 by pulling the obturator 12 in a direction opposite to a direction in which the shaft 15 is stuck into the body wall BW and leaves the cannula 11 alone at the body wall BW. As understood from the above description, the tapered portion 33 can be referred to as a palm-wrapped portion as well. In addition, the tapered portion 33 can be referred to as a curved surface portion as well.

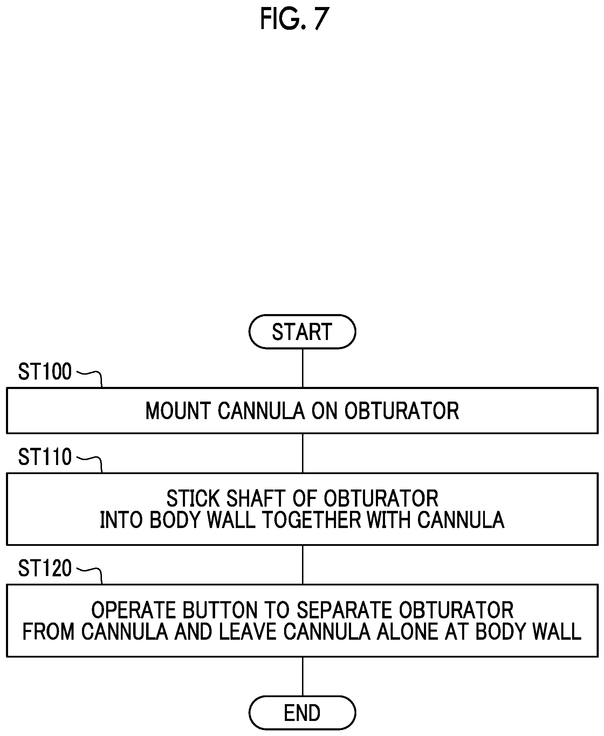

Fig. 7 is a flowchart showing an example of the procedure for operating the trocar 10. First, the operator mounts the cannula 11 on the obturator 12 (Step ST100). In this case, the operator adjusts the positions of the cannula 11 and the obturator 12 in the circumferential direction CD such that the protrusions 28 are fitted into the grooves 21 and the button 20 is accommodated in the notched portion 27.

Next, as shown in Fig. 5, the operator hooks fingers F on the recessed portions 24 of the grip 16 and sticks the shaft 15 into the body wall BW together with the cannula 11 while gripping the grip 16 (Step ST110).

After the shaft 15 is stuck into the body wall BW up to a position where the connecting portion 14 reaches the surface of the body wall BW, the operator operates the button 20 as shown in Fig. 6 so that the obturator 12 is separated from the cannula 11 and the cannula 11 is left alone at the body wall BW (Step ST120). Thereafter, the operator performs, for example, introduction of an endoscope and/or a treatment tool into the insertion holes 17 and 18 of the cannula 11 and performs surgery on the patient.

As described above, the obturator 12 includes the shaft 15, the grip 16, and the recessed portions 24. The shaft 15 is inserted into the cannula 11, and the distal end 22 thereof is sharp-pointed in a needle-like shape to be stuck into the body wall BW. The proximal end 23 of the shaft 15 is attached to the grip 16 and the grip 16 is gripped by the operator. Two recessed portions 24 are formed at the grip 16, and the fingers F of the operator are hooked on the recessed portions 24. Therefore, the obturator 12 can be further improved in terms of operation stability in comparison with an obturator disclosed in JP2003-047618Ain which only one recessed portion is formed at one surface of a grip.

As shown in Fig. 1 and the like, the two recessed portions 24 are formed at opposite positions on the grip 16. Therefore, the grip 16 can be formed in a simple and symmetrical shape.

As shown in Fig. 3 and the like, each of the recessed portion 24 includes the first surface 30 that is formed along the axial direction AD of the shaft 15 and the second surface 31 that is connected to the first surface 30 on the connection end 25 side, the connection end 25 being one end of the grip to which the proximal end 23 of the shaft 15 is attached. The second surface 31 intersects the first surface 30. With the first surface 30, a force that rotates the shaft 15 in the circumferential direction CD can be efficiently transmitted in a case where the shaft 15 is stuck into the body wall BW. In addition, with the second surface 31, a force that pushes the shaft 15 in a direction in which the shaft 15 is stuck can be efficiently transmitted in a case where the shaft 15 is stuck into the body wall BW.

As shown in Fig. 3 and the like, the first surface 30 and the second surface 31 are connected to each other by the connection surface 32 which is a curved surface. Therefore, it is possible to smoothly apply, with one finger, a force to the first surface 30 that rotates the shaft 15 in the circumferential direction CD and a force to the second surface 31 that pushes the shaft 15 in a direction in which the shaft 15 is stuck in comparison with a case where the first surface 30 and the second surface 31 are connected to each other at an angle. In addition, it is possible to prevent dust from accumulating in a connection portion between the first surface 30 and the second surface 31 in comparison with a case where the first surface 30 and the second surface 31 are connected at an angle. Furthermore, it is possible to easily clean the connection portion between the first surface 30 and the second surface 31 in comparison with a case where the first surface 30 and the second surface 31 are connected at an angle. Therefore, it is possible to maintain the cleanliness of the connection portion between the first surface 30 and the second surface 31.

As shown in Fig. 1 and the like, the grip 16 has a tapered shape (the tapered portion 33) of which the thickness decreases toward the open end 26 from the connection end 25. Since this shape is a shape that conforms to a gripping shape of the hand H, it is easy to grip the grip 16 with the hand H. Particularly, in the case of a gripping method in which the tapered portion 33 is wrapped with a palm as shown in Fig. 6, it is possible to fully exhibit an effect of being easily gripped by the hand H.

As shown in Fig. 4, the grip 16 has a shape obtained in a case where a portion of the prolate spheroid PS is cut along the short axial direction SAD. In this case, the open end 26 of the grip 16 has a rounded shape (the tapered portion 33) and thus there is no burden imposed on the palm of the hand H even in a case where the palm comes into contact with the open end 26.

As shown in Fig. 4, the length LGAD of the grip 16 in the axial direction AD is 1.2 times or more and less than 1.5 times the length LGRD of the grip 16 in the radial direction RD. In a case where the length LGAD is 1.2 times or more and less than 1.5 times the length LGRD, it is easy to grip the grip 16 with the hand H and to operate the obturator 12 (the trocar 10). On the contrary, in a case where the length LGAD is less than 1.2 times or 1.5 times or more the length LGRD, it is difficult to grip the grip 16 with the hand H and to operate the obturator 12 (trocar 10).

In addition, as shown in Fig. 4, the length LGAD of the grip 16 in the axial direction AD is 40 mm or greater and smaller than 70 mm. In a case where the length LGAD is 40 mm or greater and less than 70 mm, it is easy to grip the grip 16 with the hand H and to operate the obturator 12 (trocar 10). On the contrary, in a case where the length LGAD is less than 40 mm or 70 mm or more, it is difficult to grip the grip 16 with the hand H and to operate the obturator 12 (trocar 10).

As shown in Fig. 1, the grip 16 includes the notched portion 27 in which at least a portion of the button 20 for separation of the cannula 11 is accommodated and that is disposed at a position different from the positions of the recessed portions 24 in the circumferential direction CD. Therefore, the recessed portions 24 and the notched portion 27 do not interfere with each other and thus it is possible to reduce a probability that the button 20 is erroneously operated by the fingers F hooked on the recessed portions 24.

The grip 16 includes the protrusions 28 that are connected to the cannula 11 and that are provided at positions corresponding to the recessed portions 24 in the circumferential direction CD. Therefore, the protrusions 28 can be easily visually recognized in a case where the cannula 11 is to be mounted on the obturator 12, and it is possible to easily adjust the positions of the cannula 11 and the obturator 12 in the circumferential direction CD such that the protrusions 28 are fitted into the grooves 21.

As shown in Fig. 1 and the like, the cannula 11 includes the cannula body 13 that has a tubular shape with the insertion hole 17 into which the shaft 15 is inserted and the connecting portion 14 to which the proximal end 19 of the cannula body 13 is attached and to which the grip 16 is attachably and detachably connected. As shown in Fig. 3, the length LCAD of the connecting portion 14 in the axial direction is shorter than the length LGAD of the grip 16 in the axial direction AD. Therefore, it is possible to operate the trocar 10 with the grip 16 having a size relatively larger than the size of the connecting portion 14 and to improve the operation stability in comparison with the case of the grip 16 having a size relatively smaller than the size of the connecting portion 14.

The button 20 for separation of the cannula 11 from the obturator 12 may be provided at the grip 16 instead of the connecting portion 14. In addition, the notched portion 27 may not be provided.

Modification examples of the grip will be described below. Note that, hereinafter, parts different from the above-described embodiments will be mainly described, and the same parts as those of the above-described embodiment or parts that do not need to be distinguished from each other are given the same reference numerals as those of the above-described embodiment and the description thereof will be omitted.

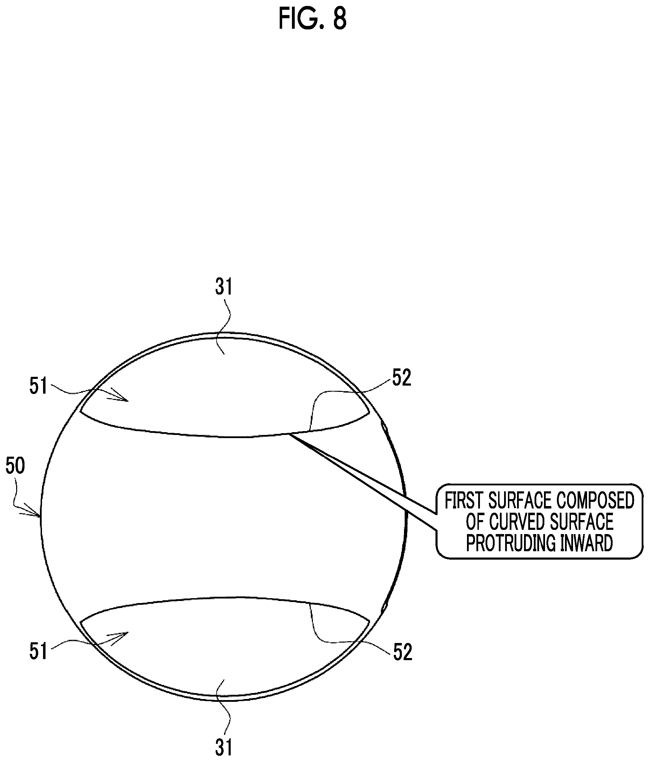

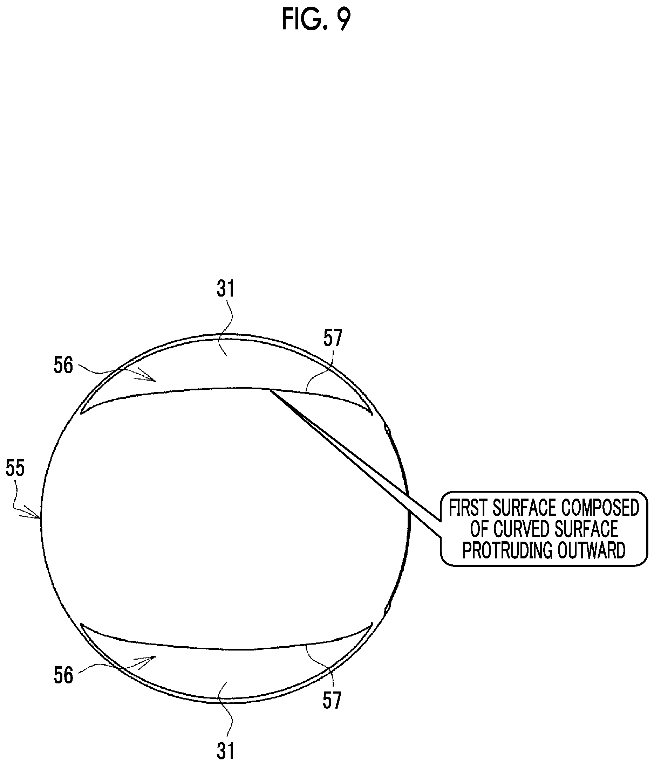

For example, a recessed portion 51 of a grip 50 shown in Fig. 8 includes a first surface 52 composed of a curved surface protruding inward. On the contrary, for example, a recessed portion 56 of a grip 55 shown in Fig. 9 includes a first surface 57 composed of a curved surface protruding outward. In the case of the recessed portion 51 shown in Fig. 8, the feeling of fitting of the finger F can be enhanced since the shape of the first surface 52 coincides with the shape of the pad of the finger F. On the other hand, in the case of the recessed portion 56 shown in Fig. 9, since the first surface 57 has a shape that further conforms to the gripping shape of the hand H, it is easier to grip the first surface 57 with the hand H.

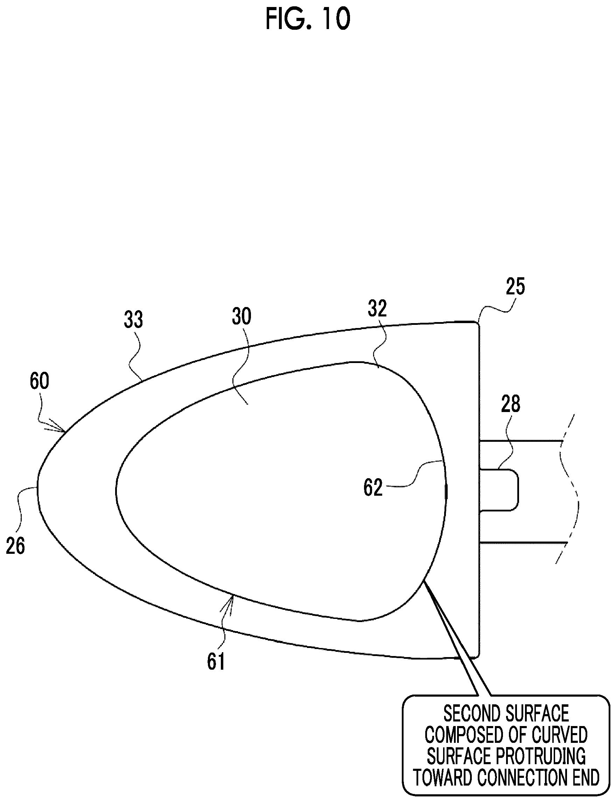

In Figs. 8 and 9, the recessed portions 51 and 56 of which the first surfaces 52 and 57 are composed of curved surfaces are shown. However, the present disclosed technology is not limited thereto. For example, as in the case of a recessed portion 61 of a grip 60 shown in Fig. 10, a second surface 62 composed of a curved surface protruding toward the connection end 25 may also be provided. In the case of the recessed portion 61, since the second surface 62 coincides with the shape of a fingertip, the feeling of fitting of the finger F can be enhanced. Note that, a second surface that is composed of a curved surface protruding toward the open end 26 unlike the second surface 62 may also be adopted. As described above, at least one of the first surface or the second surface may include a curved surface.

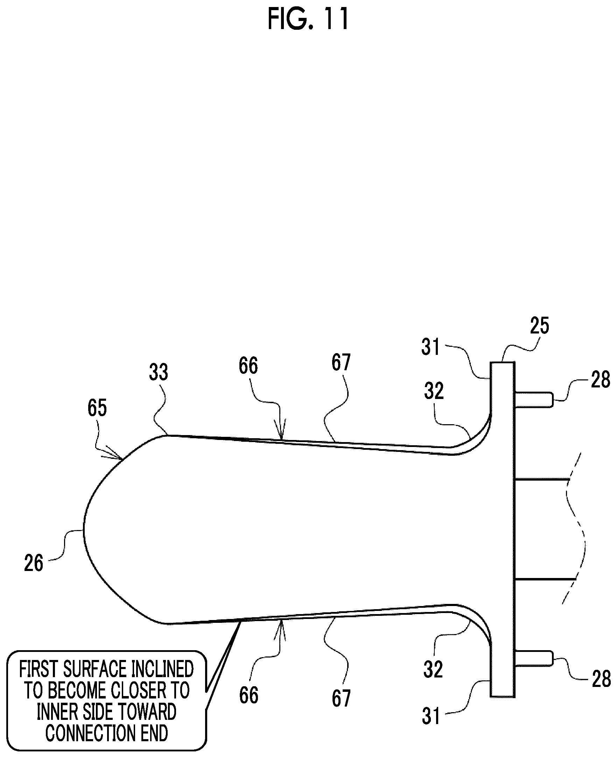

For example, a recessed portion 66 of a grip 65 shown in Fig. 11 includes a first surface 67 that is inclined to become closer to an inner side toward the connection end 25. In the case of the recessed portion 66, since the first surface 67 has a shape that further conforms to the gripping shape of the hand H, it is easier to grip the first surface 67 with the hand H. As described above, the first surface may not be a surface approximately parallel to the axial direction AD. The same applies to the second surface. In short, the first surface and the second surface only need to intersect each other.

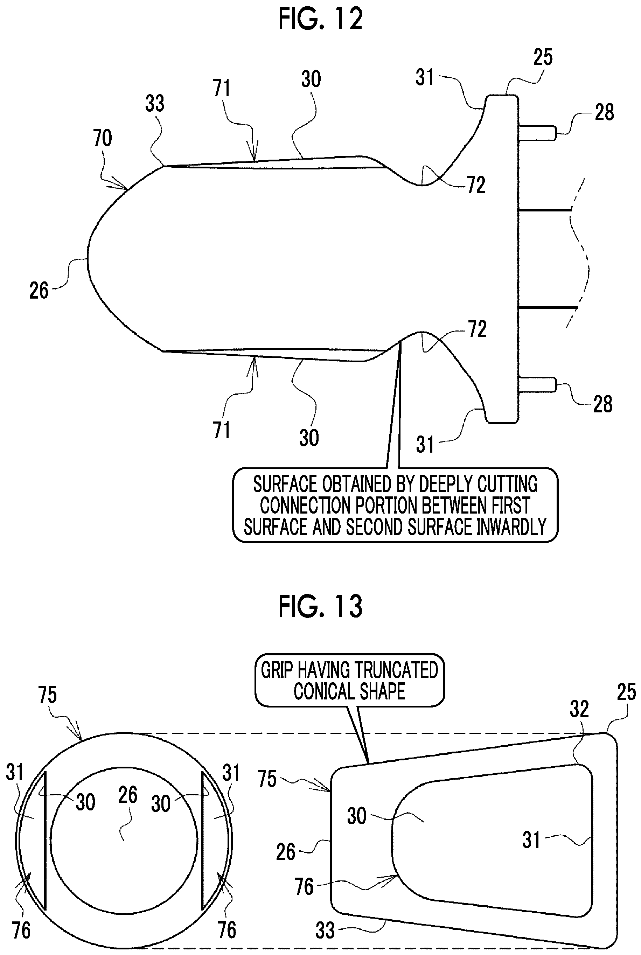

In addition, for example, a recessed portion 71 of a grip 70 shown in Fig. 12 includes a deep cut surface 72. The deep cut surface 72 is a surface obtained by deeply cutting a connection portion between the first surface 30 and the second surface 31 inwardly. The deep cut surface 72 is an example of a "curved surface" according to the present disclosed technology. In the case of the recessed portion 71, it is possible to efficiently transmit a force by means of various gripping methods such as a method of gripping the deep cut surface 72 with a fingertip put on the deep cut surface 72 or a method of gripping the deep cut surface 72 with an index finger and a thumb hooked on the deep cut surface 72.

Hereinabove, a shape obtained in a case where a portion of the prolate spheroid PS is cut along the short axial direction SAD has been used as an example of the shape of the grip. However, the present disclosed technology is not limited thereto.

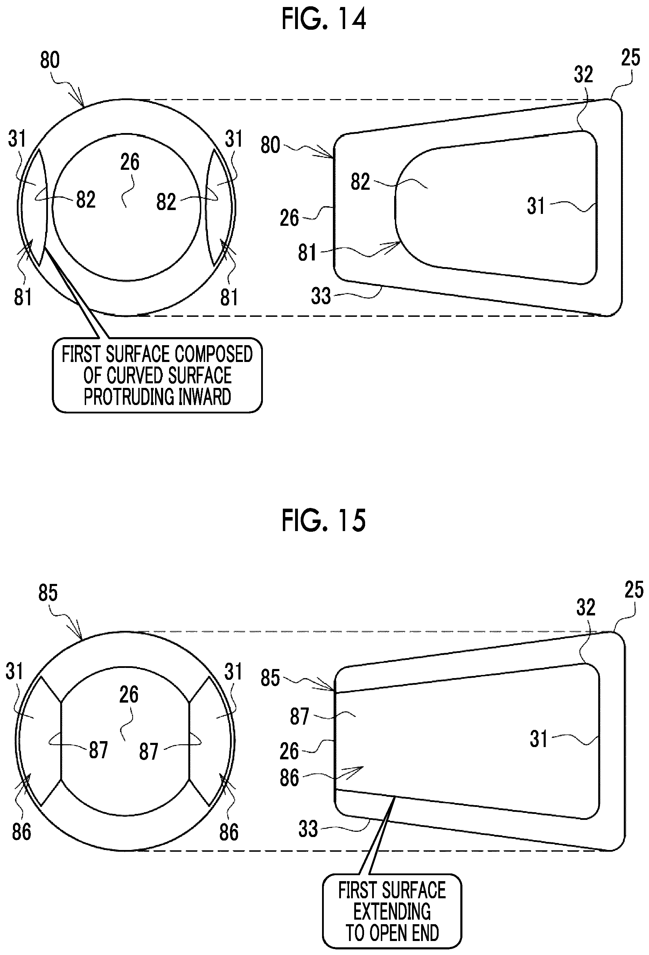

As in the case of a grip 75 shown in Fig. 13 as an example, a grip 80 shown in Fig. 14 as an example, and a grip 85 shown in Fig. 15 as an example, the grip may have a truncated conical shape. A truncated cone is a three-dimensional shape obtained by cutting a cone along a plane parallel to a bottom surface and removing a small cone portion and a pudding is a representative example thereof. Since the truncated conical shape is also a shape that conforms to a gripping shape of the hand H, it is easy to grip the grips 75, 80, and 85 with the hand H. Regarding the grips 75, 80, and 85 each having a truncated conical shape also, it is possible to fully exhibit an effect of being easily gripped by the hand H in the case of a gripping method in which the tapered portion 33 is wrapped with a palm as shown in Fig. 6. Note that, as with the grip 16, corners of the grips 75, 80, and 85 are also rounded through R chamfering.

As with the grip 16, a recessed portion 76 of the grip 75 shown in Fig. 13 includes the first surface 30, the second surface 31, and the connection surface 32. A recessed portion 81 of the grip 80 shown in Fig. 14 includes a first surface 82 composed of a curved surface protruding inward. Note that a first surface composed of a curved surface protruding outward like the first surface 57 shown in Fig. 9 may also be adopted instead of the first surface 82. In addition, a second surface composed of a curved surface protruding toward the connection end 25 like the second surface 62 shown in Fig. 10 may also be adopted instead of the second surface 31. Furthermore, a first surface that is inclined to become closer to the inner side toward the connection end 25 like the first surface 67 shown in Fig. 11 may also be adopted and a first surface and a second surface may be connected to each other by a curved surface like the deep cut surface 72 shown in Fig. 12.

A recessed portion 86 of a grip 85 shown in Fig. 15 includes a first surface 87 extending from a position near the connection end 25 to the open end 26. In the case of the first surface 87, it is easy to slide the finger F into the recessed portion 86.

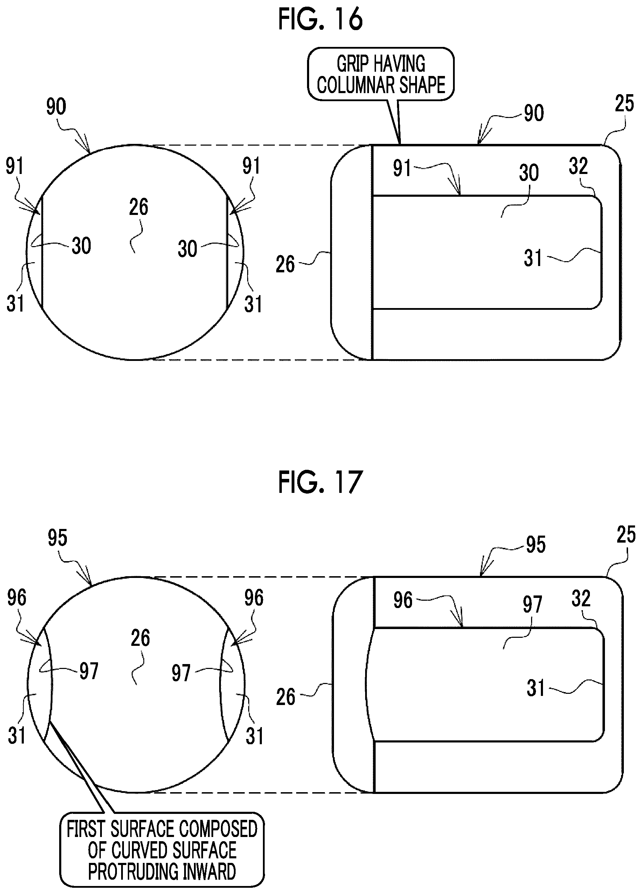

As in the case of a grip 90 shown in Fig. 16 as an example and a grip 95 shown in Fig. 17 as an example, the grip may have a columnar shape. The grips 90 and 95 each having a columnar shape can be easily manufactured in comparison with the case of a shape that is obtained in a case where a portion of the prolate spheroid PS is cut along the short axial direction SAD and a truncated conical shape. Note that, as with the grip 16 and the like, corners of the grips 90 and 95 are also rounded through R chamfering.

As with the grips 16 and 75, a recessed portion 91 of the grip 90 shown in Fig. 16 includes the first surface 30, the second surface 31, and the connection surface 32. A recessed portion 96 of the grip 95 shown in Fig. 17 includes a first surface 97 composed of a curved surface protruding inward. Note that a first surface composed of a curved surface protruding outward like the first surface 57 shown in Fig. 9 may also be adopted instead of the first surface 97. In addition, a second surface composed of a curved surface protruding toward the connection end 25 like the second surface 62 shown in Fig. 10 may also be adopted instead of the second surface 31. Furthermore, a first surface that is inclined to become closer to the inner side toward the connection end 25 like the first surface 67 shown in Fig. 11 may also be adopted and a first surface and a second surface may be connected to each other by a curved surface like the deep cut surface 72 shown in Fig. 12. In addition, a first surface that extends from a position near the connection end 25 to the open end 26 like the first surface 87 shown in Fig. 15 may also be adopted.

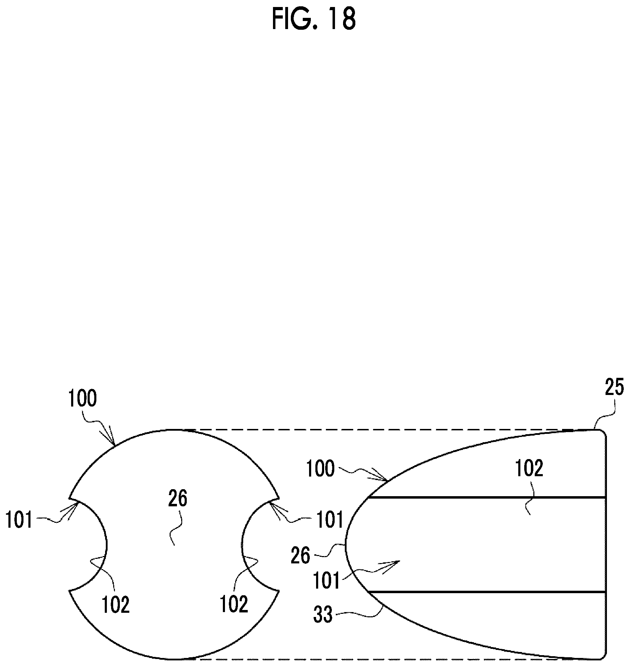

The second surface may not be provided. For example, as in the case of a recessed portion 101 of the grip 100 shown in Fig. 18, the recessed portion may include only a C-shaped first surface 102 that extends from the connection end 25 to the open end 26.

The shape of the grip may be, for example, a polygonal frustum shape such as a quadrangular frustum shape, or a polygonal prism shape such as a quadrangular prism shape. In such cases, the recessed portion further includes, in addition to the first surface and the second surface, a third surface that intersects the first surface and the second surface. In addition, the number of the recessed portions is not limited to two. For example, three recessed portions may be formed at portions 120° separated from each other in a plan view of the grip as seen in the axial direction AD.

Regarding the present disclosed technology, the above-described various embodiments and/or various modification examples can be combined with each other as appropriate. In addition, it is a matter of course that the present disclosed technology is not limited to the above-described embodiment and various configurations can be adopted without departing from the gist.

Contents described and illustrated above are for detailed description of a part according to the present disclosed technology and are merely an example of the present disclosed technology. For example, description of the above-described configurations, functions, actions, and effects is description related to an example of configurations, functions, actions, and effects of a part according to the present disclosed technology. Therefore, it is a matter of course that an unnecessary part of the contents described and illustrated above may be deleted, a new element may be added, and replacement may be made without departing from the spirit of the present disclosed technology. In addition, in order to avoid complication and facilitate the understanding of a portion according to the present disclosed technology, regarding the contents described and illustrated above, description related to common technical knowledge or the like which does not need to be described to enable implementation of the present disclosed technology has been omitted.

In the present specification, the term "A and/or B" is synonymous with the term "at least one of A or B". That is, the term "A and/or B" means only A, only B, or a combination of A and B. In addition, in the present specification, the same approach as "A and/or B" is applied to a case where three or more matters are represented by connecting the matters with "and/or".

## Claims

1. An obturator (12) comprising:
a shaft (15) that is inserted into a cannula (11) and of which a distal end is sharp-pointed in a needle-like shape to be stuck into a body wall;
a grip (16) to which a proximal end of the shaft is attached and that is gripped by an operator; and
at least two recessed portions (24) that are formed at the grip and on which fingers of the operator are hooked, wherein the two recessed portions are formed at opposite positions on the grip;
wherein the recessed portion includes at least
a first surface (30) that is formed along an axial direction of the shaft, and
a second surface (31) that is connected to the first surface (30) on a connection end side (25) and that intersects the first surface, the connection end being one end of the grip to which the proximal end of the shaft is attached, wherein the first surface and the second surface are connected to each other by a curved surface (32);
and **characterised in that** the grip (16) has a tapered shape of which a thickness decreases toward an open end from a connection end, the connection end being one end of the grip to which the proximal end of the shaft is attached and the open end being the other end of the grip that is on a side opposite to the connection end;
and wherein the grip (16) has a shape that is obtained in a case where a portion of a prolate spheroid is cut along a short axial direction or has a truncated conical shape.

2. The obturator according to claim 1,
wherein at least one of the first surface (30) or the second surface (31) includes a curved surface.

3. The obturator according to claim 1 or 2,
wherein the first surface (30) extends from a position near the connection end to an open end, which is the other end of the grip (16) and is on a side opposite to the connection end.

4. The obturator according to any one of claims 1 to 3,
wherein the grip (16) has a columnar shape.

5. The obturator according to any one of claims 1 to 4,
wherein a length of the grip in an axial direction of the shaft is 1.2 times or more and less than 1.5 times a length of the grip in a radial direction of the shaft.

6. The obturator according to any one of claims 1 to 5,
wherein a length of the grip in an axial direction of the shaft is 40 mm or greater and smaller than 70 mm.

7. The obturator according to any one of claims 1 to 6,
wherein the grip (16) includes a notched portion in which at least a portion of a button for separation of the cannula is accommodated and that is disposed at a position different from a position of the recessed portion in a circumferential direction of the shaft.

8. The obturator according to any one of claims 1 to 7,
wherein the grip includes a protrusion that is connected to the cannula and that is provided at a position corresponding to the recessed portion in a circumferential direction of the shaft.

9. A trocar comprising:
the obturator according to any one of claims 1 to 8; and
a cannula into which the obturator is attachably and detachably inserted.

10. The trocar according to claim 9,
wherein the cannula includes
a cannula body that has a tubular shape with an insertion hole into which the shaft is inserted, and
a connecting portion to which a proximal end of the cannula body is attached, to which the grip is attachably and detachably connected, and of which a length in an axial direction of the shaft is shorter than a length of the grip in the axial direction.

## Patentansprüche

1. Obturator (12), umfassend:
einen Schaft (15), der in eine Kanüle (11) eingeführt wird und dessen distales Ende zum Einstechen in eine Körperwand nadelförmig spitz ausgebildet ist,
einen Griff (16), an dem ein proximales Ende des Schafts angebracht ist und der von einem Bediener gegriffen wird; und
mindestens zwei ausgesparte Abschnitte (24), die an dem Griff gebildet sind und an denen Finger des Bedieners eingehakt sind, wobei die zwei ausgesparten Abschnitte an gegenüberliegenden Positionen an dem Griff gebildet sind;
wobei der ausgesparte Abschnitt mindestens enthält:
eine erste Fläche (30), die entlang einer Axialrichtung des Schafts gebildet ist, und
eine zweite Fläche (31), die mit der ersten Fläche (30) auf einer Verbindungsendseite (25) verbunden ist und die die erste Fläche schneidet, wobei das Verbindungsende ein Ende des Griffs ist, an dem das proximale Ende des Schafts angebracht ist, wobei die erste Fläche und die zweite Fläche durch eine gekrümmte Fläche (32) miteinander verbunden sind;
und **dadurch gekennzeichnet, dass**
der Griff (16) eine sich verjüngende Form aufweist, deren Dicke von einem Verbindungsende zu einem offenen Ende hin abnimmt, wobei das Verbindungsende ein Ende des Griffs ist, an dem das proximale Ende des Schafts angebracht ist, und das offene Ende das andere Ende des Griffs ist, das sich auf einer Seite gegenüber dem Verbindungsende befindet;
und wobei der Griff (16) eine Form aufweist, die in einem Fall erhalten wird, in dem ein Abschnitt eines prolaten Sphäroids entlang einer kurzen Axialrichtung abgeschnitten wird, oder eine kegelstumpfförmige Form aufweist.

2. Obturator nach Anspruch 1,
wobei mindestens eine von der ersten Fläche (30) und der zweiten Fläche (31) eine gekrümmte Fläche enthält.

3. Obturator nach Anspruch 1 oder 2,
wobei sich die erste Fläche (30) von einer Position nahe dem Verbindungsende zu einem offenen Ende erstreckt, das das andere Ende des Griffs (16) ist und sich auf einer Seite gegenüber dem Verbindungsende befindet.

4. Obturator nach einem der Ansprüche 1 bis 3,
wobei der Griff (16) eine säulenförmige Form aufweist.

5. Obturator nach einem der Ansprüche 1 bis 4,
wobei eine Länge des Griffs in einer Axialrichtung des Schafts das 1,2-fache oder mehr und weniger als das 1,5-fache einer Länge des Griffs in einer Radialrichtung des Schafts beträgt.

6. Obturator nach einem der Ansprüche 1 bis 5,
wobei eine Länge des Griffs in einer Axialrichtung des Schafts 40 mm oder größer und kleiner als 70 mm beträgt.

7. Obturator nach einem der Ansprüche 1 bis 6,
wobei der Griff (16) einen gekerbten Abschnitt enthält, in dem mindestens ein Abschnitt einer Taste zum Trennen der Kanüle aufgenommen ist und der an einer Position, die sich von einer Position des ausgesparten Abschnitts in einer Umfangsrichtung des Schafts unterscheidet, angeordnet ist.

8. Obturator nach einem der Ansprüche 1 bis 7,
wobei der Griff einen Vorsprung enthält, der mit der Kanüle verbunden ist und der an einer Position, die dem ausgesparten Abschnitt in einer Umfangsrichtung des Schafts entspricht, vorgesehen ist.

9. Trokar, umfassend:
den Obturator nach einem der Ansprüche 1 bis 8; und
eine Kanüle, in die der Obturator anbringbar und abnehmbar eingeführt wird.

10. Trokar nach Anspruch 9,
wobei die Kanüle enthält:
einen Kanülenkörper, der eine rohrförmige Form mit einem Einführloch aufweist, in das der Schaft eingeführt wird, und
einen Verbindungsabschnitt, an dem ein proximales Ende des Kanülenkörpers angebracht ist, mit dem der Griff anbringbar und abnehmbar verbunden ist und dessen Länge in einer Axialrichtung des Schafts kürzer als eine Länge des Griffs in der Axialrichtung ist.

## Revendications

1. Obturateur (12) comprenant :
une tige (15) qui est insérée dans une canule (11) et dont une extrémité distale est pointue en forme d'aiguille pour être enfoncée dans une paroi corporelle,
une poignée (16) à laquelle une extrémité proximale de la tige est fixée et qui est saisie par un opérateur ; et
au moins deux parties en retrait (24) qui sont formées sur la poignée et sur lesquelles des doigts de l'opérateur sont accrochés, où les deux parties en retrait sont formées à des positions opposées sur la poignée ;
dans lequel la partie en retrait inclut au moins
une première surface (30) qui est formée le long d'une direction axiale de la tige, et
une deuxième surface (31) qui est connectée à la première surface (30) sur un côté d'extrémité de connexion (25) et qui croise la première surface, l'extrémité de connexion étant une extrémité de la poignée à laquelle l'extrémité proximale de la tige est fixée, où la première surface et la deuxième surface sont connectées l'une à l'autre par une surface courbe (32) ;
et **caractérisé en ce que**
la poignée (16) a une forme effilée dont une épaisseur diminue vers une extrémité ouverte à partir d'une extrémité de connexion, l'extrémité de connexion étant une extrémité de la poignée à laquelle l'extrémité proximale de la tige est fixée et l'extrémité ouverte étant l'autre extrémité de la poignée qui se trouve sur un côté opposé à l'extrémité de connexion ;
et dans lequel la poignée (16) a une forme qui est obtenue dans un cas où une partie d'un sphéroïde allongé est coupée le long d'une direction axiale courte ou a une forme conique tronquée.

2. Obturateur selon la revendication 1,
dans lequel au moins l'une de la première surface (30) ou de la deuxième surface (31) inclut une surface courbe.

3. Obturateur selon la revendication 1 ou la revendication 2,
dans lequel la première surface (30) s'étend depuis une position proche de l'extrémité de connexion jusqu'à une extrémité ouverte, qui est l'autre extrémité de la poignée (16) et se trouve sur un côté opposé à l'extrémité de connexion.

4. Obturateur selon l'une quelconque des revendications 1 à 3,
dans lequel la poignée (16) a une forme colonnaire.

5. Obturateur selon l'une quelconque des revendications 1 à 4,
dans lequel une longueur de la poignée dans une direction axiale de la tige est de 1,2 fois ou plus et inférieure à 1,5 fois une longueur de la poignée dans une direction radiale de la tige.

6. Obturateur selon l'une quelconque des revendications 1 à 5,
dans lequel une longueur de la poignée dans une direction axiale de la tige est de 40 mm ou plus et inférieure à 70 mm.

7. Obturateur selon l'une quelconque des revendications 1 à 6,
dans lequel la poignée (16) inclut une partie crantée dans laquelle au moins une partie d'un bouton de séparation de la canule est logée et qui est disposée à une position différente d'une position de la partie en retrait dans une direction circonférentielle de la tige.

8. Obturateur selon l'une quelconque des revendications 1 à 7,
dans lequel la poignée inclut une saillie qui est connectée à la canule et qui est prévue à une position correspondant à la partie en retrait dans une direction circonférentielle de la tige.

9. Trocart comprenant :
l'obturateur selon l'une quelconque des revendications 1 à 8 ; et
une canule dans laquelle l'obturateur est inséré de manière attachable et détachable.

10. Trocart selon la revendication 9,
dans lequel la canule inclut
un corps de canule qui a une forme tubulaire avec un orifice d'insertion dans lequel la tige est insérée, et
une partie de connexion à laquelle une extrémité proximale du corps de canule est fixée, à laquelle la poignée est connectée de manière attachable et détachable, et dont une longueur dans une direction axiale de la tige est plus courte qu'une longueur de la poignée dans la direction axiale.
